# FASCICULE DE BREVET EUROPEEN

(11) **EP 1 680 139 B1**
(45) Date de publication et mention de la délivrance du brevet: **07.03.2007**
(21) Numéro de dépôt: 04791488.2
(22) Date de dépôt: 08.10.2004
(51) Int. Cl.: A61K 38/31, A61K 38/16, A61P 15/08

(54) **UTILISATION DE LA SOMATOSTATINE OU D'UN DE SES ANALOGUES POUR PREPARER UN MEDICAMENT DESTINE A REGULER LA RESERVE FOLLICULAIRE OVARIENNE CHEZ LA FEMME NON MENOPAUSEE**
VERWENDUNG VON SOMATOSTATIN ODER EINEM DER ANALOGA ZUR HERSTELLUNG EINES MEDIKAMENTS ZUR REGULIERUNG DER EIERSTOCK-FOLLIKEL-RESERVE IN NICHT-MENOPAUSALEN FRAUEN
USE OF SOMATOSTATIN OR ONE OF THE ANALOGS THEREOF FOR PRODUCING A MEDICAMENT SERVING TO REGULATE THE OVARIAN FOLLICULAR RESERVE IN NON-MENOPAUSAL WOMEN

(30) Priorité: 10.10.2003 EP 03292505
(43) Date de publication de la demande: 19.07.2006
(73) Titulaire: SOCIETE DE CONSEILS DE RECHERCHES ET D'APPLICATIONS SCIENTIFIQUES (S.C.R.A.S.), 75016 Paris (FR); INSTITUT NATIONAL DE LA SANTE ET DE LA RECHERCHE MEDICALE (INSERM), 75013 Paris (FR)
(72) Inventeur: GOUGEON, Alain, F-69630 Chaponost (FR); LOUMAYE, Ernest, F-74140 Massongy (FR)
(74) Mandataire: Bourgouin, André
(86) Numéro de dépôt international: PCT/FR2004/002536
(87) Numéro de publication internationale: WO 2005/034989

(56) Documents cités:
- WO-A-01/96393
- WO-A-02/10192
- WO-A-97/01579
- WO-A-02/072602
- DATABASE MEDLINE [Online] US NATIONAL LIBRARY OF MEDICINE (NLM), BETHESDA, MD, US; janvier 2001 (2001-01), NILSSON E ET AL: "Cellular interactions that control primordial follicle development and folliculogenesis." XP002290490 Database accession no. NLM11223364 & JOURNAL OF THE SOCIETY FOR GYNECOLOGIC INVESTIGATION. 2001 JAN-FEB, vol. 8, no. 1 Suppl Proceedings, janvier 2001 (2001-01), pages S17-S20, ISSN: 1071-5576
- DATABASE BIOSIS [Online] BIOSCIENCES INFORMATION SERVICE, PHILADELPHIA, PA, US; 1989, MACLACHLAN V ET AL: "A CONTROLLED STUDY OF LHRH AGONIST BUSERELIN FOR THE INDUCTION OF FOLLICULOGENESIS BEFORE IN-VITRO FERTILIZATION" XP002290491 Database accession no. PREV198988030508 & NEW ENGLAND JOURNAL OF MEDICINE, vol. 320, no. 19, 1989, pages 1233-1237, ISSN: 0028-4793
- DATABASE EMBASE [Online] ELSEVIER SCIENCE PUBLISHERS, AMSTERDAM, NL; 2000, DE VET A ET AL: "Regulation of early follicle development" XP002290492 Database accession no. EMB-2000168667 & TIJDSCHRIFT VOOR FERTILITEITSONDERZOEK 2000 NETHERLANDS, vol. 14, no. 1, 2000, pages 2-9, ISSN: 0921-7304
- DATABASE BIOSIS [Online] BIOSCIENCES INFORMATION SERVICE, PHILADELPHIA, PA, US; juin 1998 (1998-06), VAN DER MEER M ET AL: "Octreotide, a somatostatin analogue, alters ovarian sensitivity to gonadotrophin stimulation as measured by the follicle stimulating hormone threshold in polycystic ovary syndrome" XP002290493 Database accession no. PREV199800353993 cité dans la demande & HUMAN REPRODUCTION (OXFORD), vol. 13, no. 6, juin 1998 (1998-06), pages 1465-1469, ISSN: 0268-1161
- REUBI J C ET AL: "A new peptidic somatostatin agonist with high affinity to all five somatostatin receptors" EUROPEAN JOURNAL OF PHARMACOLOGY, AMSTERDAM, NL, vol. 456, 2002, pages 45-49, XP002276363 ISSN: 0014-2999 cité dans la demande

## Description

L'invention concerne l'utilisation de la somatostatine ou d'un de ses analogues agonistes pour préparer un médicament destiné à réguler la réserve folliculaire ovarienne chez la femme non ménopausée, ou celle d'un analogue antagoniste de la somatostatine pour préparer un médicament destiné à accélérer l'entrée en croissance des follicules quiescents chez la femme non ménopausée. L'invention concerne encore l'utilisation *in vitro* de la somatostatine ou d'un de ses analogues agonistes pour inhiber l'entrée en croissance de follicules quiescents dans un tissu ovarien, ou l'utilisation *in vitro* d'un analogue antagoniste de la somatostatine pour accélérer l'entrée en croissance des follicules quiescents dans un tissu ovarien.

Chez la femme, comme chez tous les mammifères, la fertilité est dépendante de la présence dans les ovaires des gamètes femelles appelés "ovocytes". Dans l'espèce humaine, le capital ovocytaire est constitué une fois pour toutes à la naissance ; le nombre d'ovocytes est alors compris entre 500 000 et 1 million par ovaire. Ces ovocytes sont entourés de quelques cellules de la granulosa ; cet ensemble fonctionnel est appelé follicule ovarien (Gougeon, A., *Endocrine Reviews* (1996), **17**, 121-155). A la naissance, mais aussi tout au long de la vie jusqu'à la ménopause, la majorité des follicules ovariens sont à un stade de repos.

Dès sa constitution, le capital ovocytaire s'amenuisera progressivement : ainsi, il y aura environ 200 000 follicules par ovaire à la puberté, environ 80 000 à 20 ans, environ 30 000 à 30 ans, environ 10 000 à 40 ans, le capital étant pratiquement épuisé vers l'âge de 50 ans (cf. Gougeon, A. et Lefèvre, B., « Folliculogénèse et maturation ovocytaire » dans *Médecine de la reproduction,* 3^{e} édition, Ed. Flammarion, p. 49). L'épuisement du capital ovocytaire correspond cliniquement à la ménopause. Les follicules au repos présents dans l'ovaire à un moment donné constituent la "réserve ovarienne".

Deux mécanismes sont impliqués dans l'épuisement progressif de la réserve ovarienne. Environ 80% des follicules disparaîtront par entrée en apoptose, tandis que les 20% restants entreront en croissance. Ces derniers entreprendront alors un long processus de développement (environ 6 mois) menant une minorité d'entre eux (environ 400 pendant toute la vie) au stade de follicules préovulatoires contenant un ovocyte mature apte à être fécondé (Gougeon, A., *Endocrine Reviews* (1996), **17**, 121-155). La majorité des follicules en croissance disparaissent par apoptose conduisant à leur involution; l'apoptose les frappe à n'importe quel stade de leur développement.

Le passage du stade follicule quiescent au stade de follicule en croissance est un phénomène continu mais d'intensité variable. En particulier, il s'accélère dans les 10 à 15 années précédant la ménopause, dès l'âge d'environ 38 ans.

Les facteurs stimulant les premières étapes de croissance (à partir du grand follicule primaire) sont relativement bien connus. Ils incluent les gonadotrophines (LH et FSH) mais surtout des facteurs de croissance et des stéroïdes tels que les androgènes (Machlachlan, V., New England J. of Med., 320, 1233-1237; De V et A., Tijdschrift voor Fertiliteitsonderzoek, 14, 2-9). Par contre, les mécanismes contrôlant l'initiation de la croissance folliculaire sont mal connus. Il est bien établi que cette étape de la folliculogénèse n'est pas dépendante des gonadotrophines (LH et FSH) (cf. par exemple Bullun, S. et Adashi, E., *Williams Textbook of Endocrinology,* Tenth Edition (2003), 587-664). Une hormone nommée AMH (Anti-Mullerian hormone) pourrait être impliquée dans le maintien en quiescence des follicules tandis qu'un peptide nommé Kit-Ligand (aussi appelé SCF) pourrait être impliqué dans l'activation des follicules au repos vers la croissance (Nilsson E., J. of the Soc. for Gyn. Invest., 8, S17-S20). En outre, un facteur de croissance nommé GDF-9 semble important pour soutenir la croissance une fois enclenchée (cf. par exemple WO 01/96393).

La somatostatine (SST) est un peptide cyclique présent sous deux formes dans l'organisme, une forme contenant 14 acides aminés et une forme contenant 28 acides aminés. L'activité biologique de ces deux formes de SST est semblable. La forme SST-14 est la forme prédominante au niveau du système nerveux central. Elle inhibe la sécrétion de l'hormone de croissance par les cellules somatotropes de l'anté-hypophyse. La forme SST-28 est préférentiellement exprimée au niveau de l'estomac et du pancréas. L'activité biologique de la SST est induite par l'intermédiaire d'une série de récepteurs membranaires couplés à une protéine G, dont 5 sous types ont été caractérisés, à savoir les sous-types SSTR1 à SSTR5 (Reubi, J.C., *Cancer Res.,* **47**, 551-558 ; Resine, T., et coll., *Endocr. Review,* **16**, 427- 442 ; Lamberts, SW. et coll., *Endocr. Review,* **12**, 450-482).

La présence de SST au niveau de l'ovaire a été démontrée chez plusieurs espèces dont le porc (Mori, T. et coll., *Acta Endocrinol. (Copenh.)* (1984), **106**(2), 254-259), le rat (McNeill, D.L. et coll., *Am. J. Anat.* (1987), **179**(3), 269-76) et la femme (Holst et coll., *Hum. Reprod.* (1994), **9**(8), 1448-1451). Les récepteurs à la SST ont été identifiés dans l'ovaire du rat (Lidor, A. et coll., *Gynecol. Endocrinol.* (1998), **12**(2), 97-101) ainsi que dans l'ovaire humain en particulier les sous types 1, 2A et 5 (Strauss et coll., *Hum. Reprod* (2003), **18**, Suppl. 1, P-495).

La contribution de la SST dans la physiologie ovarienne a été étudiée par plusieurs auteurs. Chez le rat, l'administration *in vivo* de SST semble réduire le nombre de cellules hypophysaires produisant LH et FSH ainsi que le nombre de follicules pré-ovulatoires dans l'ovaire (Nestorovic et coll., *Histochem. J.* (2001), **33**(11-12), 695-702). *In vitro,* la SST inhibe l'aromatase et la production de progestérone, stimulées par FSH, dans un modèle de cellules de la granulosa (Andreani, C.L. et coll., *Hum. Reprod.* (1995), **10**(8), 1968-1973). Chez le porc, la SST inhibe l'augmentation de l'AMPc induite par LH et la forskoline dans les cellules de la granulosa (Rajkumar, K. et coll., *J. Endocrinol.* (1992), **134**(2), 297-306), et semble inhiber la maturation nucléaire de l'ovocyte préovulatoire (Mori, T. et coll., *Acta Endocrinol. (Copenh.)* (1985), **110**(3), 408-412). Chez la femme, des études *in vitro* sur des cellules de la granulosa issues de follicules pré-ovulatoires suggèrent un rôle inhibiteur direct de la SST sur la synthèse de l'IGF-BP1 et de la progestérone (Holst, N. et coll., *Fertil. Steril.* (1997), **68**(3), 478-482). Chez la femme, *in vivo,* SST est capable de réduire la sécrétion de LH par l'hypophyse, de diminuer la production d'androgènes et les taux sériques de l'IGF-1. Par contre, SST augmente les taux sériques de l'IGF-BP3 (Fulghesu, A.M. et coll., *Fertil. Steril.* (1995), **64**(4), 703-708; Piaditis, G.P. et coll., *Clin. Endocrinol. (Oxf.)* (1996), **45**(5), 595-604). La SST a été co-administrée avec de la FSH lors de traitement d'induction de l'ovulation chez des patientes présentant une infertilité résultant d'un syndrome des ovaires polykystiques. La capacité de la SST à diminuer les taux sériques de LH, et à diminuer les taux sériques d'hormone de croissance et d'IGF-I a été confirmée. Cet effet endocrinien ne s'est pas cependant pas accompagné d'un impact significatif sur la croissance folliculaire induite par l'administration de FSH (Lidor, A. et coll., *Gynecol. Endocrinol.* (1998), **12**(2), 97-101; van der Meer, M. et coll., *Hum. Reprod.* (1998), **13**(6), 1465-1469). En résumé, il a été rapporté à ce jour un effet marginal de la SST sur la sécrétion hypophysaire de LH et sur la production de progestérone par les cellules de la granulosa des follicules pré-ovulatoires.

Les Demanderesses ont maintenant découvert de façon surprenante que la SST et ses analogues ont la capacité d'inhiber le passage des follicules d'un stade quiescent à un stade de croissance et que cette faculté ouvrait à ces composés de nouvelles utilisations thérapeutiques.

L'intérêt de cette découverte réside en premier lieu dans l'opportunité d'utiliser la SST ou un analogue agoniste de la SST pour préparer un médicament destiné à diminuer, voire inhiber l'entrée en croissance des follicules au stade quiescent. En second lieu, l'on pourra aussi utiliser un analogue antagoniste de ce peptide pour préparer un médicament destiné à accélérer l'entrée en croissance des follicules quiescents.

Il existe en effet un ensemble de situations cliniques pour lesquelles il serait médicalement souhaitable pour la patiente de ralentir l'utilisation de la réserve ovarienne afin de différer l'épuisement de cette dernière et donc de préserver la fonction ovarienne et la fertilité. Ces situations sont typiquement, et de manière non exclusive, les patientes à risque de ménopause précoce. Il est en effet bien connu que certaines patientes présentent un épuisement prématuré de leur capital folliculaire. La ménopause survient alors avant l'âge de 40 ans et parfois même avant l'âge de trente ans. Il est souvent possible de prévoir cette ménopause précoce sur la base d'antécédents familiaux, ou d'anomalies génétiques telles que le syndrome de Turner (complet ou partiel). Dans cette situation, l'administration de SST ou d'un de ses analogues agonistes sera préventive et aura pour but de ralentir l'entrée en croissance des follicules quiescents.

Il en est de même pour les patientes présentant une difficulté à concevoir et dont l'âge chronologique ou l'âge biologique de leurs ovaires correspond à la période d'accélération de la mobilisation des follicules quiescents : ralentir cet épuisement du capital folliculaire devrait permettre d'augmenter l'efficacité des traitements et l'opportunité d'obtenir une grossesse.

Une autre situation clinique pouvant bénéficier d'un traitement par la SST ou par un de ses analogues agonistes est la greffe (préférablement autologue) d'ovaire ou de fragments d'ovaire. Dans ce contexte, la reprise de la fonction ovarienne est souvent transitoire et s'accompagne d'une épuisement précoce du nombre de follicules primordiaux (Baird, D.T. et coll., *Endocrinology* (1999), **140**, 462-471). Il a en effet été démontré que, lors de la transplantation, les cellules de la granulosa des follicules en croissance sont plus disposées à entreprendre un phénomène d'apoptose que celle des follicules primordiaux (Liu, J. et coll., *Hum. Reprod.* (2002), **17**, 605-611). De plus, le prélèvement de tissu ovarien et leur fragmentation entraînent une mobilisation massive et rapide des follicules primordiaux vers un stade de follicules primaires tardifs (cf. Wandji S-A, et al., *Hum. Reprod.* (1997), **12**, 1993-2001; cf. également le groupe contrôle de l'exemple de la présente demande).

Dans le cadre de cette application, des analogues agonistes de la somatostatine (ou la somatostatine) pourront être ajoutés aux divers milieux de prélèvement, lavage, conservation, congélation et décongélation du tissu ovarien en vue de greffes. L'invention concerne donc également les milieux correspondants comprenant un analogue agoniste de la somatostatine. Elle concerne de même l'utilisation d'un analogue agoniste de la somatostatine comme adjuvant protecteur pour les milieux de prélèvement, lavage, conservation, congélation et décongélation du tissu ovarien.

Par ailleurs, l'utilisation *in vitro* de somatostatine ou d'analogues agonistes de la somatostatine pourra également être utile dans le domaine des analyses toxicologiques et dans la production *in vitro* d'ovocytes matures à partir de tissu ovarien frais ou congelé. Pour les premières, lors de tests de l'effet de nouvelles entités chimiques sur les follicules ovariens et leur croissance, l'ajout à l'échantillon folliculaire d'un analogue agoniste de la somatostatine permettra de ralentir la croissance folliculaire et ainsi d'observer plus facilement un effet éventuel d'accélération de ladite croissance occasionné par lesdites nouvelles entités chimiques. L'ajout d'antagoniste de la somatostatine permettra de mobiliser la réserve folliculaire vers les phases de croissance et de mieux évaluer l'impact de nouvelles entités chimiques sur ce phénomène. Pour la production *in vivo* d'ovocytes matures, l'ajout à l'échantillon folliculaire d'un analogue agoniste de la somatostatine permettra de ralentir la croissance folliculaire durant la phase initiale de mise en culture. L'ajout d'antagoniste de la somatostatine dans un second temps permettra de mobiliser la réserve folliculaire vers les phases de croissance permettant l'obtention d'un nombre supérieur de follicules matures et donc d'ovocytes fertilisables.

L'utilisation d'analogues agonistes de la somatostatine (ou la somatostatine) chez des patientes ayant des ovaires polykystiques présenterait également un intérêt. En effet, de nombreuses observations suggèrent que les ovaires polykystiques possèdent une population folliculaire anormalement élevée (cf. Hughesdon, *Obstet. Gynecol. Surv.* (1982), **37**(2), 59-77). La production d'androgènes en excès par ces follicules en surnombre pourrait être à l'origine des désordres métaboliques et endocriniens observés chez ces patientes. De plus, une diminution significative de cette population folliculaire par résection ovarienne, cautérisation ou ultra-violets, constitue l'une des thérapeutiques les plus efficaces puisqu'elle permet aux patientes d'ovuler à environ 80% et de devenir enceintes à 75% en taux cumulé sur 18 mois. Ainsi, l'administration régulière d'un analogue agoniste de la somatostatine (ou de somatostatine) devrait entraîner une diminution du nombre de follicules en croissance et donc des follicules antraux surnuméraires producteurs d'androgènes ayant pour conséquence la reprise de cycles ovulatoires fertiles de façon transitoire ou permanente.

Par ailleurs, l'utilisation d'analogues agonistes de la somatostatine (ou de somatostatine) chez des patientes sur le point de subir, subissant ou ayant subi une chimiothérapie ou une irradiation (à visée thérapeutique ou autre) réduira le risque de ménopause précoce en prévenant la mobilisation accélérée de la réserve folliculaire qui la rendrait plus sensible aux agents de la chimiothérapie ou aux rayonnements ionisants.

D'autres applications peuvent encore être envisagées, en particulier dans le domaine vétérinaire. L'invention pourrait être mise au service de la sauvegarde des espèces, l'utilisation d'analogues agonistes de la somatostatine (ou de somatostatine) permettant de préserver la réserve ovarienne des femelles. De même, des analogues antagonistes de la somatostatine peuvent être utilisés dans le cadre de fécondations *in vitro* ou *in vivo* chez animaux de haute valeur commerciale. De tels animaux de haute valeur commerciale peuvent notamment être des chevaux, des bovins, des ovins ou des caprins ; il peut également s'agir d'animaux d'origine transgénique.

En dehors des pathologies évoquées précédemment, un ralentissement systématique de l'épuisement de la réserve ovarienne pourrait être envisagé chez la femme ne souffrant d'aucun dysfonctionnement ovarien. Dans les pays industrialisés, l'allongement continu de l'espérance de vie (actuellement environ 83 ans en France) s'accompagne d'un allongement de la période post-ménopausique et des troubles qui y sont associés : cardiopathies, ostéoporose, vieillissement cutané, etc. Des doutes apparaissent quant à l'innocuité à long terme des traitements hormonaux substitutifs de la ménopause. En conséquence, une alternative élégante consisterait à décaler l'âge de survenue de la ménopause. Cela réduirait d'autant la période postménopausique et les risques associés. Ce décalage ne signifierait pas pour autant que la fertilité pourrait être maintenue jusqu'à 60 ans et plus. En effet de nombreux travaux suggèrent que la fonction ovarienne est maintenue tant qu'un nombre minimum de follicules de la réserve est maintenu, et qu'en dépit d'une fonction ovarienne "normale" (taux de stéroïdes peu affectés), les possibilités de grossesse sont extrêmement faibles.

Dans les situations où l'on souhaite ralentir l'utilisation de la réserve ovarienne, on utilisera selon l'invention la somatostatine naturelle (SST14 ou SST28), ou, de préférence, un analogue agoniste de la somatostatine (naturel ou synthétique). L'analogue agoniste de la somatostatine peut être un polypeptide cyclique ou non cyclique, une protéine de fusion ou de recombinaison, une entité chimique non peptidique (i.e. peptidomimétique) ou encore un peptide « *SS-like* » tel que la corticostatine. Les analogues agonistes à utiliser devront présenter une haute affinité pour le récepteur SST et induire une activité fonctionnelle de celui-ci telle que l'inhibition de la sécrétion d'hormone de croissance par des cellules somatotropes de l'hypophyse et/ou l'inhibition de la prolifération in vitro de cellules d'adénome hypophysaire. De préférence, l'analogue agoniste de la somatostatine aura une haute affinité pour l'ensemble ou au moins 2 ou 3 des sous types de récepteurs de la SST ou alors une affinité privilégiée pour au moins l'un des sous-types 1, 2, 3, 4 et 5 (par exemple pour le sous-type 2).

Des analogues agonistes de la somatostatine ont été décrits notamment dans la demande de brevet PCT WO 01/00676 ou WO 98/08528 ou encore dans les brevets US 6,387,932, US 6,268,342, US 6,057,338, US 6,025,372.

Selon une variante particulière de l'invention, les analogues agonistes de la somatostatine seront des composés de formule générale **(I)** 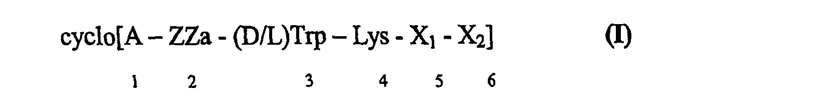 dans laquelle :
X₁ est un radical de formule (a) ou (b) 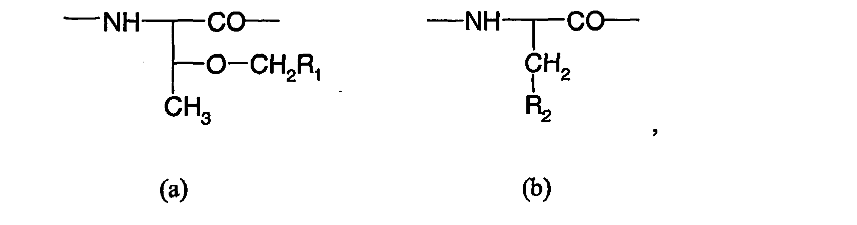 R₁ représentant indépendamment à chaque fois qu'il intervient un radical phényle éventuellement substitué dans lequel les substituants éventuels sont choisis indépendamment parmi un atome halogène et les radicaux méthyle, éthyle, méthoxy et éthoxy,
R₂ représentant -Z₁-CH₂-R₁, -CH₂-CO-O- CH₂-R₁, 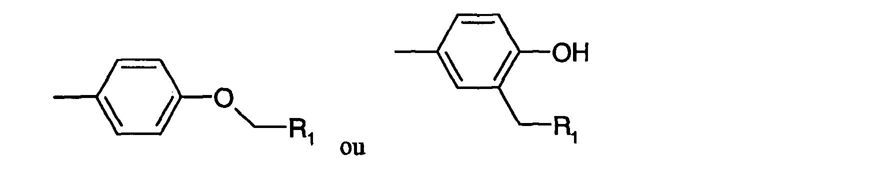
Z₁ étant O ou S ;
X₂. est un α-aminoacide ayant un résidu aromatique sur-la chaîne latérale C_{α}, ou une unité acide aminé choisie parmi Dab, Dpr, Dpm, His, (Bzl)HyPro, thiényl-Ala, cyclohexyl-Ala et t-butyl-Ala ;
A est un résidu divalent choisi parmi Pro, 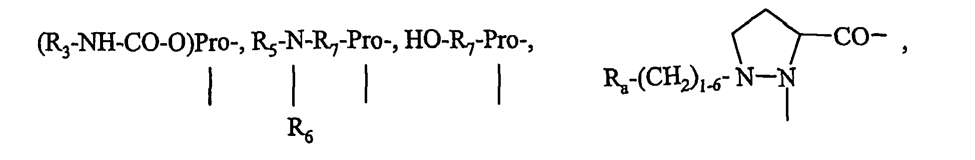 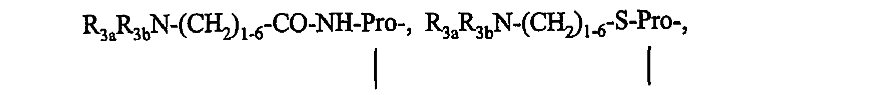 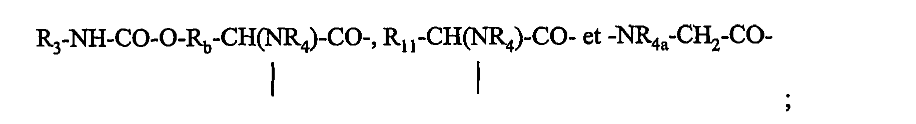
R₃ est NR₈R₉-C₂₋₆alkylène, guanidino-C₂₋₆alkylène ou C₂₋₆alkylène-COOH, R₃ₐ est H, C₁₋₄alkyle ou a indépendamment l'une des significations données pour R₃, R_{3b} est H ou C₁₋₄ alkyle, Rₐ est OH ou NR₅R₆, R_{b} est -(CH₂)₁₋₃- ou -CH(CH₃)-, R₄ est H ou CH₃, R₄ₐ est benzyle éventuellement substitué sur le cycle aromatique, chacun de R₅ et R₆ est indépendamment H, C₁₋₄alkyle, ω-amino-C₁₋₄alkylène, ω-hydroxy-C₁₋₄alkylène ou acyle, R₇ est une liaison directe ou C₁₋₆alkylène, chacun de R₈ et R₉ est indépendamment H, C₁₋₄alkyle, ω-hydroxy-C₂₋₄alkylène, acyle ou CH₂OH-(CHOH)_{c}-CH₂- dans lequel c est 0, 1, 2, 3 ou 4, ou R₈ et R₉ forment ensemble avec l'atome d'azote auquel ils sont attachés un groupe hétérocyclique qui peut comprendre un hétéroatome supplémentaire, et R₁₁ est benzyle éventuellement substitué sur le cycle aromatique, -(CH₂)₁₋₃-OH, CH₃-CH(OH)- ou -(CH₂)₁₋₅-NR₅R₆, et ZZₐ est une unité α-aminoacide naturelle ou non naturelle ;
étant entendu que X₁, X₂ et Lys ont chacun la configuration L ;
ou bien seront des sels pharmaceutiquement acceptables ou des formes protégées de composés de formule générale **(I)**.

ZZₐ peut avoir une configuration D ou L. ZZₐ peut être par exemple Thr, Ser, Ala, Val, Ile, Leu, Nle, His, Arg, Lys, Nal, Pal, Tyr, Trp, Phe substitué sur le noyau aromatique ou N^{α}-benzyl-Gly. Lorsque ZZₐ est Phe, son noyau benzénique peut être par exemple substitué par NH₂, NO₂, CH₃, OCH₃ ou un atome halogène, de préférence en position *para.* Lorsque ZZₐ est Phe, son noyau benzénique est de préférence non substitué.

Lorsque A comprend un résidu aminoacide Pro, tout substituant présent sur le cycle proline, par exemple R₃-NH-CO-O- etc., est de préférence en position 4. De tels résidus proline substitués peuvent se présenter dans la forme *cis,* par exemple 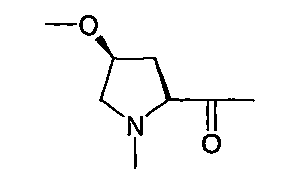 comme dans la forme *trans* form. Chaque isomère géométrique individuellement ainsi que des mélanges de ces isomères sont concernés par les utilisations selon l'invention. Lorsque A est 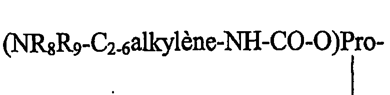 dans lequel NR₈R₉ forme un groupe hétérocyclique, ledit groupe peut être aromatique ou saturé et peut comprendre un atome d'azote ou un atome d'azote et un second hétéroatome choisi parmi l'azote et l'oxygène. De préférence, le groupe hétérocyclique est par exemple pyridyle ou morpholino. Le radical C₂₋₆alkylène dans ce résidu est de préférence -CH₂-CH₂-.

Un groupe acyle tel que R₅, R₆, R₈ et R₉ dans A peut être par exemple un groupe R₁₂CO- dans lequel R₁₂ est H, C₁₋₄alkyle, C₂₋₆alkényle, C₃₋₆cycloalkyle ou benzyle, et de méthyle ou éthyle. Lorsque R₄ₐ ou R₁₁ dans A est benzyle substitute sur le noyau aromatique, le noyau benzénique peut être substitué comme indiqué ci-dessus pour ZZₐ.

Selon une variante préférée de l'invention, les analogues agonistes de la somatostatine seront des composés de formule générale **(II)** 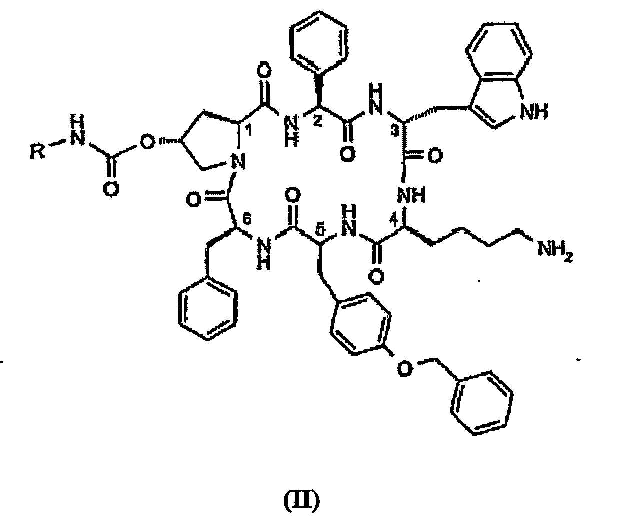 dans laquelle R est NR₁₀R₁₁-C₂₋₆alkylène ou guanidine-C₂₋₆alkylène, et chacun de R₁₀ et R₁₁ est indépendamment H ou C₁₋₄alkyle
ou bien seront des sels pharmaceutiquement acceptables ou des formes protégées de composés de formule générale **(II).**

De préférence, R sera NR₁₀R₁₁-C₂₋₆alkylène. Les composés préférés de formule générale **(II)** sont ceux tels que R est 2-aminoéthyle (et en particulier le peptide SOM 230 de formule cyclo[{4-(NH₂-C₂H₄-NH-CO-O-)Pro}-Phg-DTrp-Lys-Tyr(4-Bzl)-Phe] dont la structure est reproduite plus loin).

Par « forme protégée » d'un composé de formule générale **(I)** ou **(II),** on entend dans la présente demande un analogue de la somatostatine dans lequel au moins l'un des groupes amino est protégé et dont la déprotection (qui de préférence s'effectue d'elle-même en milieu physiologique) conduit à un composé de formule générale **(I)** ou **(II)**. Des groupes protecteurs adéquats pour groupes amino sont par exemple ceux décrits dans *Protective Groups in Organic Synthesis,* T.W. Greene, J. Wiley & Sons NY (1981), 219-287. Un exemple de tel groupe protecteur pour un groupe amino est le groupe acétyle.

Parmi les analogues agonistes de la somatostatine utilisables selon l'invention, on peut citer plus particulièrement le lanréotide, l'octréotide, le vapréotide, le SOM 230 (voir structure ci-après), le MK-678 (peptide de structure cyclo(N-Me-Ala-Tyr-D-Trp-Lys-Val-Phe)), le BIM-23190 (peptide de structure N-hydroxyéthylpipérazinyl-acétyl-D-Phe-cyclo [Cys-Tyr-D-Trp-Lys-Abu-Cys]-Thr-NH₂), le BIM-23197 (peptide de structure Hepes-D-Phe-cyclo [Cys-Tyr-D-Trp-Lys-Abu-Cys]-Thr-NH₂ dans laquelle Abu représente l'acide aminobutyrique), le BIM-23268 (peptide de structure cyclo[Cys-Phe-Phe-D-Trp-Lys-Thr-Phe-Cys]-NH₂), le PTR-3173 (voir structure ci-après), le TT-232 (de structure D-Phe-cyclo[Cys-D-Trp-Lys-Cys]-Thr-NH₂), et leurs sels pharmaceutiquement acceptables ; on peut également citer le peptide synthétique de formule c[Tic-Tyr-DTrp-Lys-Abu-Phe] et ses sels pharmaceutiquement acceptables ; on peut enfin citer le peptide KE 108 de formule Tyr⁰-(cyclo-D-Dab-Arg-Phe-Phe-D-Trp-Lys-Thr-Phe) décrit notamment dans Reubi et coll., *Eur. J. Pharmacol.* (2002), **456**, 45-49. On préférera tout particulièrement employer le lanréotide, l'octréotide ou un de leurs sels pharmaceutiquement acceptables, et plus particulièrement le lanréotide ou un de ses sels pharmaceutiquement acceptables.

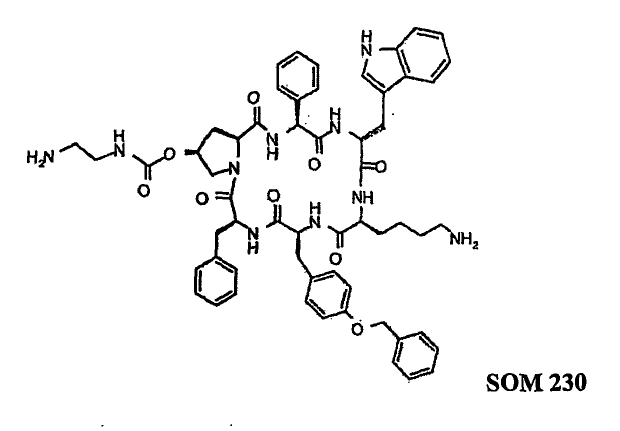

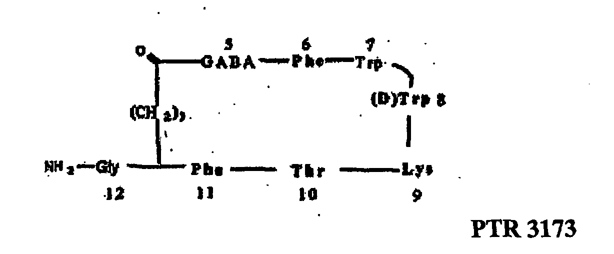

De façon analogue aux composés de formule générale **(I)** ou **(II)**, les peptides susmentionnés pourront également se présenter sous une forme protégée. Le définition de la forme protégée donnée plus haut pour les composés de formules générales **(I)** ou **(II)** est applicable *mutatis mutandis.*

Selon une variante préférée de l'invention, les patientes auxquelles sera destiné le médicament à base de somatostatine ou d'analogue agoniste de la somatostatine mentionné précédemment seront des femmes ayant un facteur de risque de ménopause précoce, et notamment des femmes ayant des antécédents familiaux de ménopause précoce. Selon une variante particulière de l'invention, les patientes auxquelles sera destiné le médicament à base de somatostatine ou d'analogue agoniste de la somatostatine mentionné précédemment seront des femmes atteintes d'une micro-délétion du chromosome X ou d'un syndrome de Turner partiel.

Le second intérêt de la découverte évoquée précédemment réside dans l'opportunité de préparer un médicament à base d'un analogue antagoniste de la somatostatine pour accélérer l'entrée en croissance des follicules quiescents. En effet, un couple sur six parmi ceux qui souhaitent une grossesse présente une difficulté à concevoir. Bien que les causes soient multiples, deux types de traitement ont émergé et sont utilisés de façon régulière en médecine humaine pour le traitement de la stérilité. Ces traitements, aussi appelés "Assistance Médicale à la Procréation" (AMP), consistent d'abord à induire la croissance simultanée de plusieurs follicules pré-ovulatoires. Ceci permet d'obtenir plusieurs ovocytes matures, et donc plusieurs embryons, et ainsi d'augmenter les chances de conception. Ceci est réalisé par l'administration d'un ou plusieurs médicaments stimulant la sécrétion hypophysaire des gonadotrophines (FSH et LH), tel qu'un anti-oestrogène (par exemple le citrate de clomiphène ou le tamoxifène) ou un inhibiteur de l'aromatase (par exemple le létrozole, fanastrazole ou l'exémestane). La croissance simultanée de plusieurs follicules pré-ovulatoires peut aussi être induite par l'administration d'une préparation de FSH humaine (extractive ou recombinante) associée ou non à de la LH. Lorsque les follicules ont atteint une taille pré-ovulatoire, selon la cause de la stérilité, deux options de traitement existent. La première est de réaliser une insémination intra-utérine (IIU) et la seconde est de prélever les ovocytes dans l'ovaire par aspiration des follicules (entre 5 et 15 ovocytes) et de réaliser une insémination au laboratoire (*in vitro*)*,* soit par simple co-incubation des ovocytes avec les spermatozoïdes du partenaire (FIV) ou par micro-injection d'un spermatozoïde directement dans l'ovocyte (ICSI). L'obtention de plusieurs ovocytes matures est critique pour optimiser les taux de succès (taux de grossesse) obtenus par ces traitements ; or chez certaines femmes, malgré une stimulation ovarienne appropriée, le nombre d'ovocytes obtenus est faible voire même égal à un. Cette difficulté à répondre au traitement stimulant résulte du nombre restreint de follicules en croissance présents dans les ovaires de ces patientes. Il est donc d'un intérêt thérapeutique important de pouvoir mobiliser des follicules de la réserve ovarienne et de les faire entrer en phase de croissance.

Un autre objet de la présente invention consiste en l'utilisation d'un analogue antagoniste de la somatostatine pour préparer un médicament destiné à accélérer l'entrée en croissance des follicules quiescents chez la femme non ménopausée.

L'administration d'un tel médicament pendant une période de 1 à 12 mois chez la femme conduira à une augmentation du nombre de follicules en phase de croissance et donc susceptibles d'être stimulés par les traitements standard pour atteindre le stade de follicules pré-ovulatoires.

Une autre application de la capacité des analogues antagonistes de la somatostatine d'induire la croissance folliculaire précoce est leur utilisation *in vitro* dans des cultures de follicules pour la production d'ovocytes matures destinés à la fécondation. L'analogue antagoniste de la somatostatine sera ajouté au milieu de culture utilisé pour soutenir le développement folliculaire *in vitro.* L'invention concerne donc également les milieux correspondants comprenant un analogue antagoniste de la somatostatine.Par ailleurs, la capacité des analogues antagonistes de la somatostatine d'induire la croissance folliculaire précoce peut aussi être utilisée dans le domaine des analyses toxicologiques. En particulier, lors de tests de l'effet de nouvelles entités chimiques sur la croissance folliculaire, l'ajout à l'échantillon folliculaire d'un analogue antagoniste de la somatostatine permettra d'accélérer la croissance folliculaire et ainsi d'observer plus facilement un effet éventuel de ralentissement de ladite croissance occasionné par lesdites nouvelles entités chimiques.

L'analogue antagoniste de la somatostatine peut être un polypeptide cyclique ou non cyclique, une protéine de fusion ou de recombinaison, une entité chimique non peptidique (i.e. peptidomimétique) ou encore un peptide « *SS-like* » tel que la corticostatine. Les analogues antagonistes à utiliser devront présenter une haute affinité pour le récepteur SST et inhiber l'activité fonctionnelle de SST14 ou SST28 telle que l'inhibition de la sécrétion d'hormone de croissance par des cellules somatotropes de l'hypophyse et/ou l'inhibition de la prolifération in vitro de cellules d'adénome hypophysaire. De préférence, l'analogue antagoniste de la somatostatine aura une haute affinité pour l'ensemble ou au moins 2 ou 3 des sous types de récepteurs de la SST ou alors une affinité privilégiée pour au moins l'un des sous-types 1, 2, 3, 4 et 5 (par exemple pour le sous-type 2).

Un analogue antagoniste de la somatostatine utilisable pour préparer selon l'invention pourra par exemple être un peptide de formule générale

A¹-cyclo {D-Cys-A²-D-Trp-A³-A⁴-Cys}-A⁵-Y¹ **(III)**

dans laquelle :
A¹ est un α-aminoacide aromatique éventuellement substitué ;
A² est un α-aminoacide aromatique éventuellement substitué ;
A³ est Dab, Dap, Lys ou Orn ;
A⁴ est β-Hydroxyvaline, Ser, Hser, ou Thr ;
A⁵ est un D- ou L- α-aminoacide aromatique éventuellement substitué ; et
Y¹ est OH, NH₂ ou NHR¹, R¹ étant (C₁₋₆)alkyle ;
chaque α-aminoacide aromatique éventuellement substitué étant éventuellement substitué avec un ou des substituants choisis indépendamment parmi le groupe composé d'un atome halogène et des groupes NO₂, OH, CN, (C₁₋₆)alkyl, (C₂₋₆)alkényle, (C₂₋₆)alkynyle, (C₁₋₆)alkoxy, Bzl, O-Bzl et NR⁹R¹⁰, R⁹ et R¹⁰ étant chacun indépendamment H, O, ou (C₁₋₆) alkyle; et
chaque atome d'azote de liaison amide peptidique et le groupe amino de A¹ étant éventuellement substitué avec un groupe méthyle, étant entendu qu'il y a au moins un tel groupe méthyle dans un peptide de formule générale **(III) ;**
ou un sel pharmaceutiquement acceptable d'un peptide de formule générale **(III).**

Par "α-aminoacide aromatique", on entend un résidu aminoacide de formule 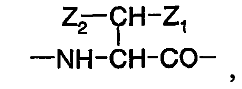 dans laquelle Z₁ est un radical contenant un cycle aromatique et Z₂ est un atome d'hydrogène ou un radical contenant un cycle aromatique. Des exemples de tels radicaux contenant un cycle aromatique incluent, mais ne sont pas limités à, un cycle benzénique ou pyridine et les structures suivantes avec ou sans un ou des substituants X sur le cycle aromatique (X étant, indépendamment à chaque fois qu'il intervient, un atome halogène, NO₂, CH₃, OCH₃, CF₃ ou OH) : 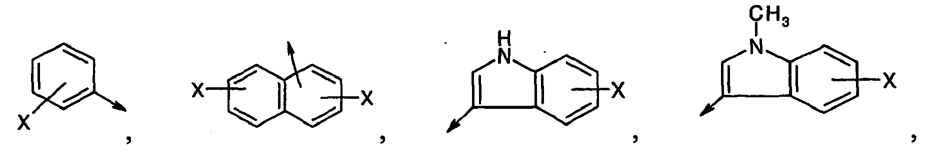 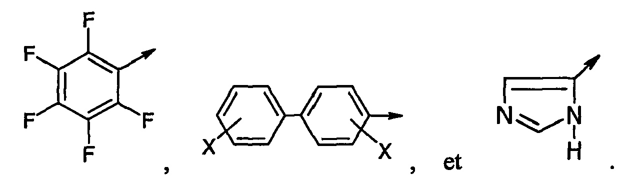 D'autres exemples d'un "α-aminoacide aromatique" selon l'invention sont des His substituées, telles que MeHis, His (τ-Me) ou His (π-Me).

D'autres analogues antagonistes de la somatostatine ont été décrits notamment dans les demandes de brevet PCT WO 98/08528, WO 98/08529, WO 98/24807, WO 98/44921, WO 98/44922, WO 98/45285 et WO 99/22735, ou encore dans les brevets US 6,387,932, US 6,262,229, US 6,063,796, US 6,057,338, US 6,025,372, US 5,925,618, US 5,846,934 et US 4,508,711.

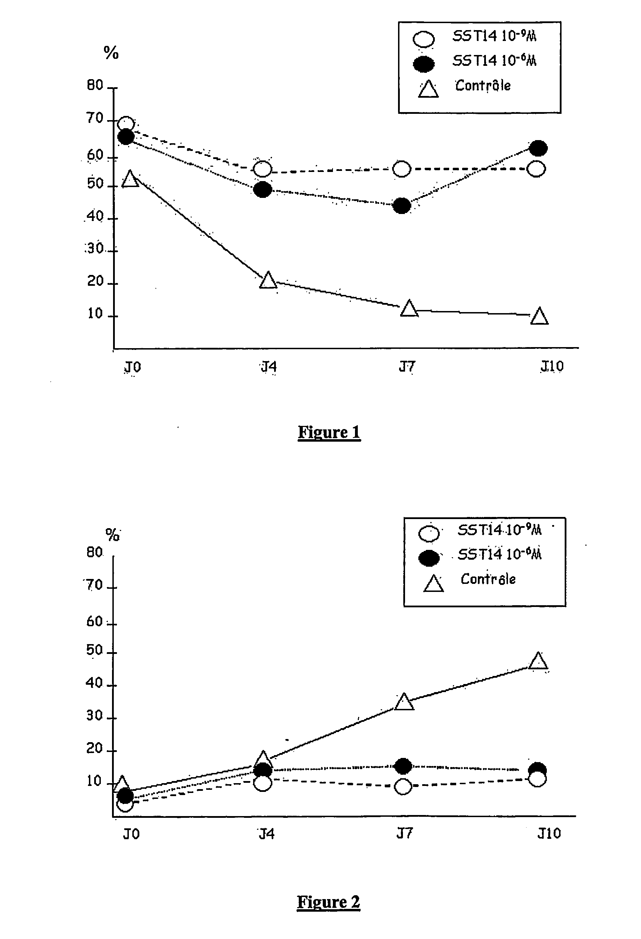

Parmi les analogues antagonistes de la somatostatine utilisables selon l'invention et leurs sels pharmaceutiquement acceptables, on pourra citer plus particulièrement :
❖ les peptides de formule générale **(III)** suivants :
   - Cpa-cyclo[D-Cys-Pal-D-Trp-N-Me-Lys-Thr-Cys]-D-Trp-NH₂ ;
   - Cpa-cyclo[D-Cys-Tyr-D-Trp- N-Me-Lys-Thr-Cys]-Nal-NH₂ ;
   - Cpa-cyclo[D-Cys-Pal-D-Trp- N-Me-Lys-Thr-Cys] Nal-NH₂ ;
❖ le peptide connu sous le nom de code AC-178,335 (de structure acétyl-D-His-D-Phe-D-Ile-D-Arg-D-Trp-D-Phe-NH₂) ;
❖ l'octapeptide connu sous le nom de code ODN-8 (cf. Fig. 1 de *Proc. Natl. Acad. Sci. USA* (2000), **97**(25), 13973-13978) ;
❖ le peptide connu sous le nom de code SB-710411 (de structure Cpa-cyclo[D-Cys-Pal-D-Trp-Lys-Val-Cys]-Cpa-amide) ;
❖ le peptide connu sous le nom de code BIM-23056 (de structure représentée ci-après) ;
❖ le composé connu sous le nom de code BN-81674 (de structure représentée ci-après) ;
❖ le composé connu sous le nom de code SRA-880 (de structure représentée ci-après) ;
et les sels pharmaceutiquement acceptables de ces derniers.

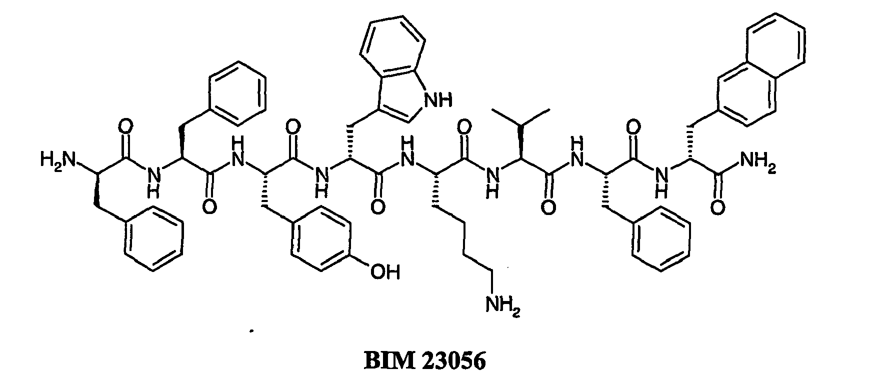

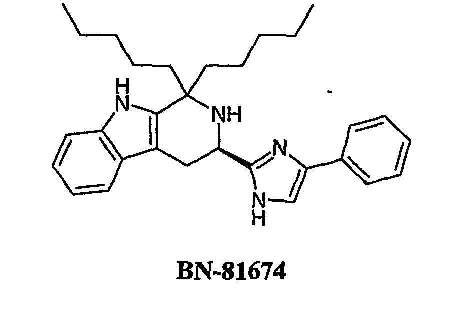

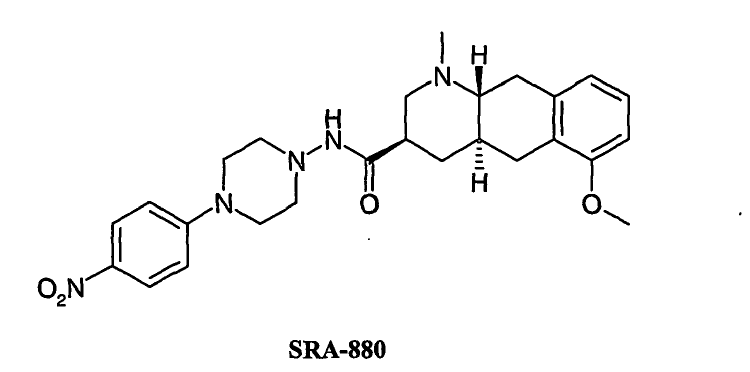

De façon analogue aux composés de formule générale **(I)** ou **(II)**, les peptides susmentionnés (y compris ceux répondant à de formule générale **(III)**) pourront également se présenter sous une forme protégée. Le définition de la forme protégée donnée plus haut pour les composés de formules générales **(I)** ou **(II)** est applicable *mutatis mutandis.*

Par analogue agoniste de la somatostatine, on entend dans la présente demande un composé pour lequel la dose effective DE₅₀ déterminée au test de l'effet agoniste décrit plus loin est inférieure ou égale à 1 µM pour au moins l'un des sous-récepteurs de la somatostatine.

Par analogue antagoniste de la somatostatine, on entend dans la présente demande un composé pour lequel la dose effective DE₅₀ déterminée au test de l'effet antagoniste décrit plus loin est inférieure ou égale à 1 µM pour au moins l'un des sous-récepteurs de la somatostatine.

Par sel pharmaceutiquement acceptable, on entend notamment dans la présente demande des sels d'addition avec des acides inorganiques tels que chlorhydrate, bromhydrate, iodhydrate, sulfate, phosphate, diphosphate et nitrate ou avec des acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt sélection for basic drugs", *Int. J. Pharm.* (1986), **33**,201-217.

Selon la présente invention, les préparations pharmaceutiques contenant de la somatostatine ou un de ses analogues agonistes ou antagonistes applicables à cette invention pourront être administrée par voie parentérale (sous cutanée, intra-musculaire, intra-péritonéale, intra-veineuse, ou en implant), par voie orale, vaginale, rectale, nasale, sublinguale ou transdermale. La voie vaginale sera préférée car elle permet de délivrer des concentrations effectives du principe actif au niveau ovarien tout en minimisant l'exposition systémique. La somatostatine ou l'analogue de la somatostatine utilisé sera formulé avec les excipients nécessaires et connus de l'homme de l'art, pour permettre une administration efficace et reproductible pour chaque voie d'administration.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Les situations typiques suivantes pour une utilisation selon l'invention pourraient toutefois être envisagées :
❖ Une patiente d'environ 20 à 25 ans (par exemple) présente un syndrome de Turner partiel par micro-délétion d'un chromosome X. Sa fonction ovarienne est apparemment normale avec des cycles ovulatoires réguliers. Son taux sérique de FSH est légèrement élevé durant la période de transition lutéo-folliculaire (par exemple FSH = environ 9,2 UI/litre). L'échographie ovarienne réalisée par voie trans-vaginale montre des ovaires de volume normaux avec un nombre de follicules antraux légèrement diminué. Considérant son risque élevé de présenter une ménopause précoce, la patiente est traitée avec de l'acétate de lanréotide à une dose de 120 mg/mois (Somatuline^{®} Autogel^{®} 120 mg, Beaufour Ipsen Pharma, France). Le traitement est interrompu après plusieurs années lorsque la patiente souhaite concevoir.
❖ Une patiente d'environ 35 à 40 ans présente une stérilité primaire depuis plusieurs années. Le bilan du couple conclu en une stérilité d'origine tubaire, résultant très probablement d'un antécédent de péritonite. Les cycles menstruels sont ovulatoires et le taux sérique de FSH est légèrement élevé durant la période de transition lutéo-folliculaire (par exemple FSH = environ 11,4 UI/L). L'échographie ovarienne réalisée par voie trans-vaginale montre des ovaires de volume légèrement diminué avec un nombre de follicules antraux diminués (environ 3 par ovaire). Un diagnostic de diminution de la réserve ovarienne est posé. Un traitement par fécondation *in vitro* est recommandé et la patiente entreprend un traitement de stimulation ovarienne par injection quotidienne de 225 unités de FSH recombinante. Au 6^{e} jour de stimulation, une échographie ovarienne montre un seul follicule en croissance de 14 mm dans l'ovaire droit. La dose de FSH est doublée et la patiente est revue 2 jours plus tard. Un seul follicule de 18 mm est observé, confirmant une diminution de la réserve ovarienne. Le traitement est interrompu. Après retour d'un cycle spontané, un traitement par administration quotidienne d'un analogue antagoniste de la somatostatine est initié. Durant ce traitement, le nombre de follicules antraux présents dans chaque ovaire est estimé par échographie en début de chaque cycle menstruel. Après 4 mois de traitement, le nombre de follicules antraux est en moyenne d'environ 6 par ovaire et la FSH sérique a diminué. Une stimulation par FSH recombinante est initiée, un développement folliculaire multiple est obtenu, et une procédure de fécondation *in vitro* classique est effectuée.
❖ Une patiente ayant un syndrome des ovaires polykystiques présente des règles très peu fréquentes, une absence d'ovulation, un poids excessif et des signes cutanés d'excès d'androgènes tels que acné et hirsutisme. A l'examen échographique du pelvis, l'endomètre est hyperplasique, les ovaires présentent un volume augmenté, un stroma augmenté, et plus de 10 follicules antraux par section ovarienne. Aucun follicule ne présente un diamètre supérieur à 10 mm. La patiente est traitée avec de l'acétate de lanréotide à une dose de 120 mg/mois (Somatuline^{®} Autogel^{®} 120 mg, Beaufour Ipsen Pharma, France). Après 3 mois de traitement, la patiente est réglée spontanément. Au 4^{e} mois de traitement, l'échographie des ovaires indique une réduction du volume ovarien et du nombre de follicules antraux. Un follicule de 16 mm est observé. Pendant les 5^{e} et 6^{e} mois de traitement, la patiente est réglée régulièrement et sa courbe de température est biphasique, suggérant une ovulation. Au 8^{e} mois de traitement, la patiente n'est pas réglée et un test de grossesse est positif. Le traitement avec l'acétate de lanréotide est interrompu.

### Abréviations et définitions particulières utilisées dans la présente demande :

Les abréviations des acides aminés courants sont en accord avec les recommandations IUPAC-IUB. Par ailleurs, les définitions pour certaines abréviations utilisées dans la présente demande sont comme suit :

| | |
|---|---|
| Abu = | acide α-aminobutyrique ; |
| Aib = | acide α-aminoisobutyrique ; |
| β-Ala = | β-alanine ; |
| Amp = | 4-amino-phénylalanine ; |
| Ava = | acide 5-aminovalérique ; |
| Bzl = | benzyle ; |
| Cha = | cyclohexylalanine ; |
| Cpa = | 3-(4-chlorophényl)alanine; |
| Dab = | acide 2,4-diaminobutyrique ; |
| Dap = | acide 2, 3-diaminopropionique ; |
| Dip = | 3, 3'-diphénylalanine ; |
| GABA = | acide γ-aminobutyrique ; |
| HSer = | homosérine ; |
| 1-Nal = | 3-(1-naphthyl)alanine ; |
| 2-Nal = | 3-(2-naphthyl)alanine ; |
| Nle = | norleucine ; |
| Nva = | norvaline ; |
| 2-Pal = | 3-(2-pyridyl)alanine ; |
| 3-Pal = | 3-(3-pyridyl)alanine ; |
| 4-Pal = | 3-(4-pyridyl)alanine ; |
| Phg = | -HN-CH(C₆H₅)-CO- |
| Tfm = | trifluorométhyle ; |
| TfmA = | 4-trifluorométhylphényl-alanine ; |
| Tic = | acide 1,2,3,4-tétrahydroisoquinoline-3-carboxylique. |

En outre, NMeLys représente la N-méthyl-lysine, dans laquelle l'azote de la liaison peptidique est méthylé (et non l'azote de la chaîne latérale de la lysine).
Enfin, Tyr(I) représente un résidu tyrosine iodé (par exemple 3-1-Tyr, 5-I-Tyr, 3,5-I-Tyr) dans lequel l'atome d'iode peut être un isotope radioactif, par exemple I₁₂₅, I₁₂₇ ou I₁₃₁.

Par ailleurs, le terme "environ" fait référence à un intervalle autour de la valeur considérée. Tel qu'utilisé dans la présente demande, "environ X" signifie un intervalle de X moins 10% de X à X plus 10% de X, et de préférence un intervalle de X moins 5% de X à X plus 5% de X.

### Préparation des peptides de formule générale (I) :

Les peptides de formules générales **(I)** et **(II)** mentionnées ci-dessus et leur synthèse sont décrits entre autres dans les demandes de brevet PCT WO 97/01579 et WO 02/10192.

Les peptides de formule générale **(III)** mentionnés ci-dessus et leur synthèse sont décrits dans la demande de brevet PCT WO 02/072602.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doit en aucun cas être considéré comme une limite à la portée de l'invention.

### EXEMPLES :

### Exemple 1 :

Des ovaires de brebis adultes sont collectés immédiatement après abattage. Les ovaires sont placés dans un milieu de transport d'organes sans sérum (X-vivo, Bio Whittaker, Walkersville, MD, USA) à 10 °C et acheminé au laboratoire. Environ 1 h après le prélèvement, le cortex est isolé de la medulla puis fractionné en tranches de 2 mm d'épaisseur (1 cm², poids moyen de 212 mg) après rinçage dans du X-vivo neuf. Les fragments de cortex sont cultivés en étuve sous 5% d'oxygène pendant 10 jours dans des boites à puits en présence de DMEM. Le milieu est changé tous les 2 jours.
Dans les fragments contrôles (incubées en l'absence de SST) les follicules primordiaux passent progressivement vers le stade de follicules en début de croissance (voir les Figures 1 et 2). L'addition de SST14 à des concentrations variant entre 10⁻⁹ M et 10⁻⁶ M inhibe très significativement l'entrée des follicules primordiaux en croissance comme le montre le maintien au cours du temps du nombre de follicules primordiaux (cf. Figure 1) et l'absence d'augmentation du nombre de follicules primaires (cf. Figure 2).

### Exemple 2

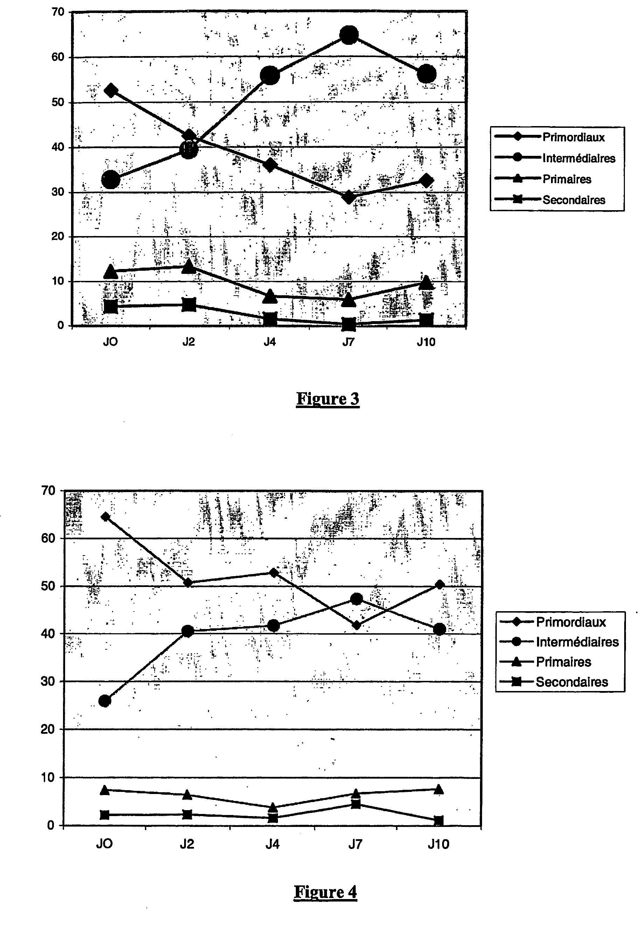

La procédure employée est la même que pour l'exemple 1, excepté que la somatostatine est remplacée par un de ses analogues agonistes, à savoir le peptide synthétique de formule c[Tic-Tyr-DTrp-Lys-Abu-Phe] (ci-après peptide AG₁).
Dans les fragments contrôles (incubées en l'absence du peptide AG₁) les follicules primordiaux passent progressivement vers le stade de follicules en début de croissance (cf. Figure 3). L'addition du peptide AG₁ à une concentration de 10⁻⁹M inhibe très significativement l'entrée des follicules primordiaux en croissance comme le montre le maintien au cours du temps du nombre de follicules primordiaux et l'absence d'augmentation du nombre de follicules primaires (cf. Figure 4).

### Exemple 3

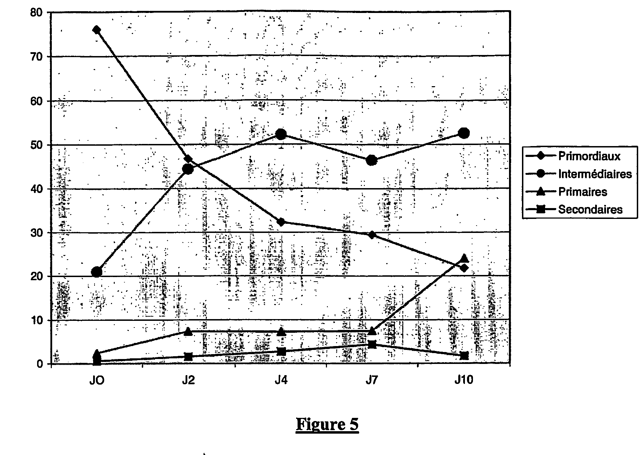

La procédure employée est la même que pour l'exemple 1, excepté que la somatostatine est remplacée par un de ses analogues antagonistes, à savoir le peptide synthétique de formule Cpa-c(DCys-3-Pal-DTrp-NMeLys-Thr-Cys)-2-Nal-NH₂ (ci-après peptide ANT₁).
Dans les fragments contrôles (incubées en l'absence du peptide ANT₁) les follicules primordiaux passent progressivement vers le stade de follicules en début de croissance (cf. Figure 3). L'on constate que l'addition du peptide ANT₁ à une concentration de 10⁻⁶ M accentue l'entrée des follicules primordiaux en croissance (cf. Figure 5).

### Brève description des figures

La **Figure 1** représente les proportions de follicules au repos au cours d'une période de 10 jours de culture de cortex ovarien de brebis en présence de somatostatine (SST14) ou non (contrôle). Ces proportions sont mesurées pour chaque échantillon testé au jour du début de l'expérience (J0), au 4^{e} jour (J4), au 7^{e} jour (J7) et au 10^{e} jour (J10).

La **Figure 2** représente les proportions de follicules primaires au cours d'une période de 10 jours de culture de cortex ovarien de brebis en présence de somatostatine (SST14) ou non (contrôle). Ces proportions sont mesurées pour chaque échantillon testé au jour du début de l'expérience (J0), au 4^{e} jour (J4), au 7^{e} jour (J7) et au 10^{e} jour (J10).

La **Figure 3** représente les proportions de follicules primordiaux, intermédiaires, primaires et secondaires au cours d'une période de 10 jours de culture de cortex ovarien de brebis en l'absence d'analogue agoniste ou antagoniste de la somatostatine. Ces proportions sont mesurées pour chaque échantillon testé au jour du début de l'expérience (J0), au 4^{e} jour (J4), au 7^{e} jour (J7) et au 10^{e} jour (J10).

La **Figure 4** représente les proportions de follicules primordiaux, intermédiaires, primaires et secondaires au cours d'une période de 10 jours de culture de cortex ovarien de brebis en présence d'un analogue agoniste de la somatostatine, le peptide synthétique de formule c[Tic-Tyr-DTrp-Lys-Abu-Phe] (peptide AG₁) ou non. Ces proportions sont mesurées pour chaque échantillon testé au jour du début de l'expérience (J0), au 4^{e} jour (J4), au 7^{e} jour (J7) et au 10^{e} jour (J10).

La **Figure 5** représente les proportions de follicules primordiaux, intermédiaires, primaires et secondaires au cours d'une période de 10 jours de culture de cortex ovarien de brebis en présence d'un analogue antagoniste de la somatostatine, le le peptide synthétique de formule Cpa-c(DCys-3-Pal-DTrp-NMeLys-Thr-Cys)-2-Nal-NH₂ (peptide ANT₁). Ces proportions sont mesurées pour chaque échantillon testé au jour du début de l'expérience (J0), au 4^{e} jour (J4), au 7^{e} jour (J7) et au 10^{e} jour (J10).

### Tests de détermination de l'effet agoniste ou antagoniste d'un analogue de la somatostatine

### Inhibition de la production intracellulaire d'AMPc

Des cellules CRO-K1 exprimant les sous-types de récepteurs de la somatostatine humaine (SRIF-14) sont cultivées dans des plaques à 24 puits dans un milieu RPMI 1640 contenant 10% de sérum foetal de veau. Le milieu est changé le jour précédant l'expérience.
Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 minutes à environ 37 °C. La production d'AMP cyclique est stimulée par l'addition de 1 mM de forskoline (FSK ; fournisseur : Sigma Chemical Co., St. Louis, MO, USA) pendant 15-30 minutes à environ 37 °C.

### Détermination de l'effet agoniste d'un analogue de la somatostatine

L'effet agoniste d'un analogue de la somatostatine est mesuré par l'addition simultanée de FSK (1 µM) et de l'analogue à tester (10⁻¹⁰) M à 10⁻⁵ M).
Le milieu réactionnel est éliminé et 200 ml de HCl 0,1 N sont ajoutés. La quantité d'AMPc est mesurée par un test radioimmunologique (Kit FlashPlate SMP001A, New England Nuclear, Boston, USA).

### Détermination de l'effet antagoniste d'un analogue de la somatostatine

L'effet antagoniste d'un analogue de la somatostatine est mesuré par l'addition simultanée de FSK (1 µM), SRIF-14 (1 à 10 nM) (fournisseur : Bachem, Torrence, CA, USA) et de l'analogue à tester (10⁻¹⁰ M à 10⁻⁵ M).
Le milieu réactionnel est éliminé et 200 ml de HC1 0,1 N sont ajoutés. La quantité d'AMPc est mesurée par un test radioimmunologique (Kit FlashPlate SMP001A, New England Nuclear, Boston, USA).

## Revendications

1. Utilisation de la somatostatine ou d'un de ses analogues agonistes pour préparer un médicament destiné à réguler la réserve folliculaire ovarienne, et en particulier à diminuer l'épuisement de la réserve folliculaire ovarienne avec le temps, chez la femme non ménopausée.

2. Utilisation selon la revendication 1, **caractérisée en ce que** la somatostatine est utilisée pour préparer le médicament.

3. Utilisation selon la revendication 1, **caractérisée en ce qu'**un analogue agoniste de la somatostatine est utilisé pour préparer le médicament.

4. Utilisation selon la revendication 3, **caractérisée en ce que** l'analogue agoniste de la somatostatine est un composé de formule générale **(I)** 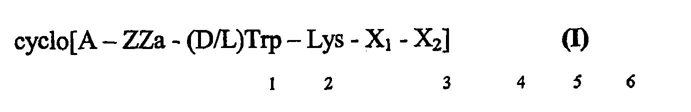 dans laquelle :
X₁ est un radical de formule (a) ou (b) 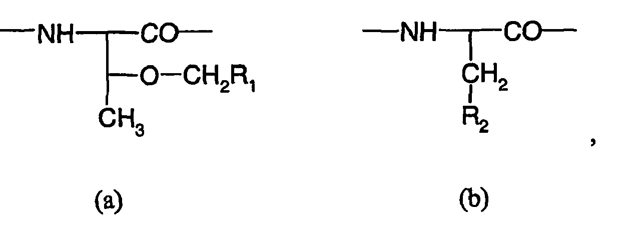
R₁ représentant indépendamment à chaque fois qu'il intervient un radical phényle éventuellement substitué dans lequel les substituants éventuels sont choisis indépendamment parmi un atome halogène et les radicaux méthyle, éthyle, méthoxy et éthoxy,
R₂ représentant Z₁-CH₂-R₁, -CH₂-CO-O- CH₂-R₁, 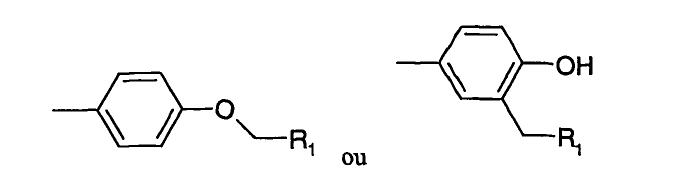
Z₁ étant O ou S ;
X₂ est un α-aminoacide ayant un résidu aromatique sur la chaîne latérale C_{α}, ou une unité acide aminé choisie parmi Dab, Dpr, Dpm, His, (Bzl)HyPro, thiényl-Ala, cyclohexyl-Ala et t-butyl-Ala ;
A est un résidu divalent choisi parmi Pro, 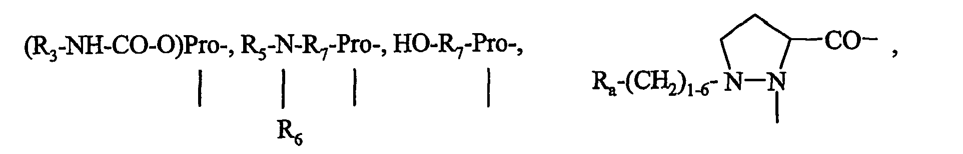 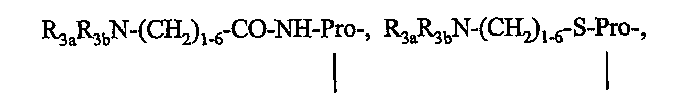 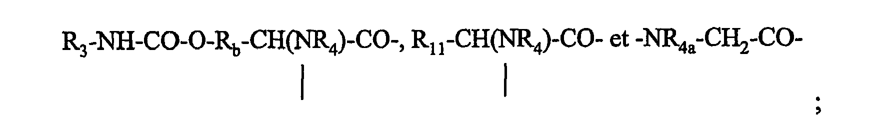
R₃ est NR₈R₉-C₂₋₆alkylène, guanidino-C₂₋₆alkylène ou C₂₋₆alkylène-COOH, R₃ₐ est H, C₁₋₄alkyle ou a indépendamment l'une des significations données pour R₃, R_{3b} est H ou C₁₋₄ alkyle, Rₐ est OH ou NR₅R₆, R_{b} est -(CH₂)₁₋₃- ou -CH(CH₃)-, R₄ est H ou CH₃, R₄ₐ est benzyle éventuellement substitué sur le cycle aromatique, chacun de R₅ et R₆ est indépendamment H, C₁₋₄alkyle, ω-amino-C₁₋₄alkylène, ω-hydroxy-C₁₋₄alkylène ou acyle, R₇ est une liaison directe ou C₁₋₆alkylène, chacun de R₈ et R₉ est indépendamment H, C₁₋₄alkyle, ω-hydroxy-C₂₋₄alkylène, acyle ou CH₂OH-(CHOH)_{c}-CH₂- dans lequel c est 0, 1, 2, 3 ou 4, ou R₈ et R₉ forment ensemble avec l'atome d'azote auquel ils sont attachés un groupe hétérocyclique qui peut comprendre un hétéroatome supplémentaire, et R₁₁ est benzyle éventuellement substitué sur le cycle aromatique, -(CH₂)₁₋₃-OH, CH₃-CH(OH)- ou -(CH₂)₁₋₅-NR₅R₆, et ZZₐ est une unité α-aminoacide naturelle ou non naturelle ;
étant entendu que X₁, X₂ et Lys ont chacun la configuration L ;
ou bien est un sel pharmaceutiquement acceptable ou une forme protégée d'un composé de formule générale **(I)**.

5. Utilisation selon la revendication 3, **caractérisée en ce que** l'analogue agoniste de la somatostatine est un composé de formule générale **(II)** 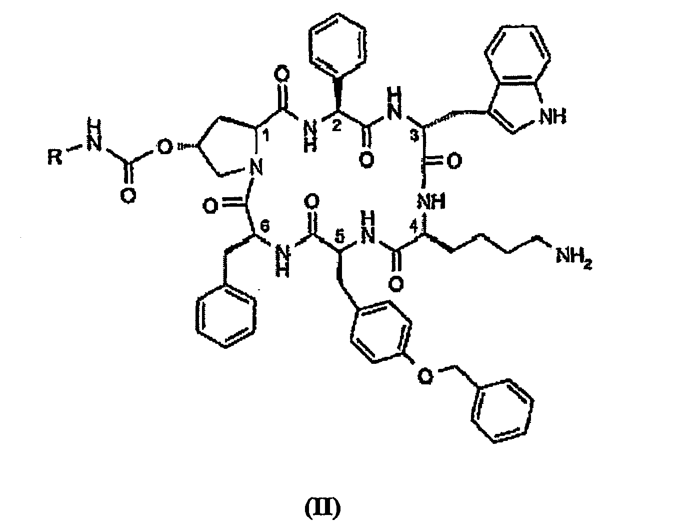 dans laquelle R est NR₁₀R₁₁-C₂₋₆alkylène ou guanidine-C₂₋₆alkylène, et chacun de R₁₀ et R₁₁ est indépendamment H ou C₁₋₄alkyle
ou bien est un sel pharmaceutiquement acceptable ou une forme protégée d'un composé de formule générale **(II)**.

6. Utilisation selon la revendication 3, **caractérisée en ce que** l'analogue agoniste de la somatostatine est choisi parmi le groupe composé du lanréotide, de l'octréotide, du vapréotide, du SOM 230, du MK-678, du BIM-23190, du BIM-23197, du BIM-23268, du PTR-3173, du TT-232, du peptide de formule c[Tic-Tyr-DTrp-Lys-Abu-Phe], du peptide KE 108 de formule Tyr⁰-(cyclo-D-Dab-Arg-Phe-Phe-D-Trp-Lys-Thr-Phe) et des sels pharmaceutiquement acceptables et formes protégées de ces derniers.

7. Utilisation selon la revendication 6, **caractérisée en ce que** l'analogue agoniste de la somatostatine est le lanréotide ou un des sels pharmaceutiquement acceptables de ce dernier.

8. Utilisation selon l'une des revendications 1 à 7, **caractérisée en ce que** le médicament est destiné à être administré à une femme ayant un facteur de risque de ménopause précoce.

9. Utilisation selon l'une des revendications 1 à 7, **caractérisée en ce que** le médicament est destiné à être administré à une femme atteinte d'une micro-délétion du chromosome X.

10. Utilisation selon l'une des revendications 1 à 7, **caractérisée en ce que** le médicament est destiné à être administré à une femme ayant des ovaires polykystiques.

11. Utilisation selon l'une des revendications 1 à 7, **caractérisée en ce que** le médicament est destiné à être administré à une femme sur le point de subir, subissant ou ayant subi une chimiothérapie ou une irradiation.

12. Utilisation de la somatostatine ou d'un de ses analogues agonistes dans des tests toxicologiques relatifs à un autre composé afin de déterminer la présence ou l'absence d'un effet d'accélération de la croissance folliculaire causée par causé par ledit autre composé.

13. Utilisation d'un analogue antagoniste de la somatostatine pour préparer un médicament destiné à accélérer l'entrée en croissance des follicules quiescents chez la femme non ménopausée.

14. Utilisation selon la revendication 13, **caractérisée en ce que** l'analogue antagoniste de la somatostatine est choisi parmi les peptides de formule générale **(III)**
A¹-cyclo{D-Cys-A²-D-Trp-A³-A⁴-Cys}-A⁵-Y¹ **(III)**
dans laquelle :
A¹ est un α-aminoacide aromatique éventuellement substitué ;
A² est un α-aminoacide aromatique éventuellement substitué ;
A³ est Dab, Dap, Lys ou Orn ;
A⁴ est β-Hydroxyvaline, Ser, Hser, ou Thr ;
A⁵ est un D- ou L-α-aminoacide aromatique éventuellement substitué ; et
Y¹ est OH, NH₂ ou NHR¹, R¹ étant (C₁₋₆)alkyle ;
chaque α-aminoacide aromatique éventuellement substitué étant éventuellement substitué avec un ou des substituants choisis indépendamment parmi le groupe composé d'un atome halogène et des groupes NO₂, OH, CN, C₁₋₆)alkyl, (C₂₋₆)alkényle, (C₂₋₆)alkynyle, (C₁₋₆)alkoxy, Bzl, O-Bzl et NR⁹R¹⁰, R⁹ et
R¹⁰ étant chacun indépendamment H, O, ou (C₁₋₆) alkyle; et
chaque atome d'azote de liaison amide peptidique et le groupe amino de A¹ étant éventuellement substitué avec un groupe méthyle, étant entendu qu'il y a au moins un tel groupe méthyle dans un peptide de formule générale **(III)** ;
et les sels pharmaceutiquement acceptables et formes protégées de tels peptides.

15. Utilisation selon la revendication 13, **caractérisée en ce que** l'analogue antagoniste de la somatostatine est choisi parmi le groupe composé :
❖ les peptides suivants :
- Cpa-cyclo[D-Cys-Pal-D-Trp-N-Me-Lys-Thr-Cys]-D-Trp-NH₂;
- Cpa-cyclo[D-Cys-Tyr-D-Trp- N-Me-Lys-Thr-Cys]-Nal-NH₂;
- Cpa-cyclo[D-Cys-Pal-D-Trp- N-Me-Lys-Thr-Cys] Nal-NH₂;
❖ le peptide connu sous le nom de code AC-178,335 ;
❖ l'octapeptide connu sous le nom de code ODN-8 ;
❖ le peptide connu sous le nom de code SB-710411 ;
❖ le peptide connu sous le nom de code BIM-23056 ;
❖ le composé connu sous le nom de code BN-81674 ;
❖ le composé connu sous le nom de code SRA-880 ;
et des sels pharmaceutiquement acceptables et formes protégées de ces derniers.

16. Utilisation d'un analogue antagoniste de la somatostatine pour soutenir le développement folliculaire *in vitro.*

17. Utilisation d'un analogue antagoniste de la somatostatine dans des tests toxicologiques relatifs à un autre composé afin de déterminer la présence ou l'absence d'un effet de ralentissement de la croissance folliculaire causé par ledit autre composé.

## Claims

1. Use of somatostatin or one of its agonist analogues for preparing a medicament intended to regulate the ovarian follicular reserve, and in particular to reduce the depletion of the ovarian follicular reserve over time, in non-menopausal women.

2. Use according to claim 1, **characterized in that** somatostatin is used for preparing the medicament.

3. Use according to claim 1, **characterized in that** a somatostatin agonist analogue is used for preparing of the medicament.

4. Use according to claim 3, **characterized in that** the somatostatin agonist analogue is a compound of general formula **(I)** 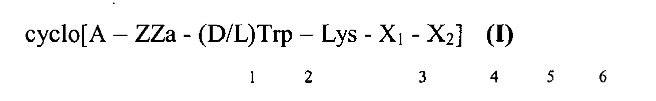 in which:
X₁ is a radical of formula (a) or (b) 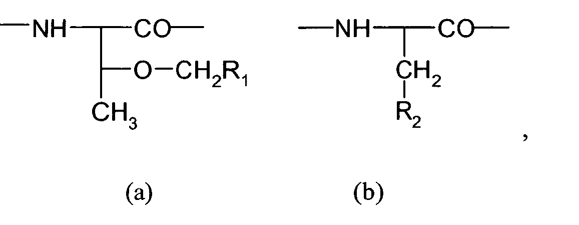
R₁ independently representing at each time that it occurs an optionally substituted phenyl radical in which the optional substituents are independently chosen from a halogen atom and the methyl, ethyl, methoxy and ethoxy radicals,
R₂ representing -Z₁-CH₂-R₁, -CH₂-CO-O- CH₂-R₁, 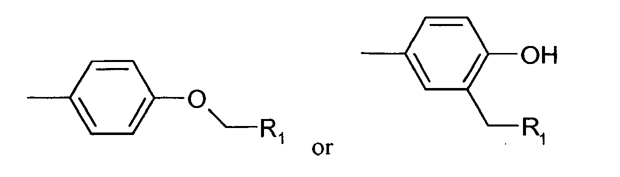
Z₁ being O or S;
X₂ is an α-amino acid having an aromatic residue on the side chain C_{α}, or an amino acid unit chosen from Dab, Dpr, Dpm, His, (Bzl)HyPro, thienyl-Ala, cyclohexyl-Ala and t-butyl-Ala;
A is a divalent residue chosen from Pro, 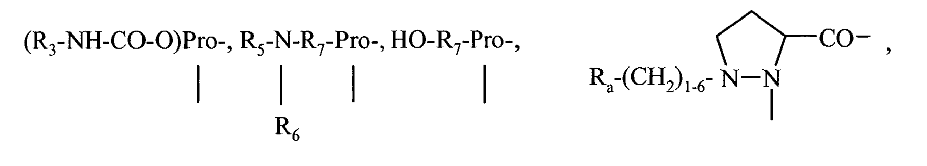 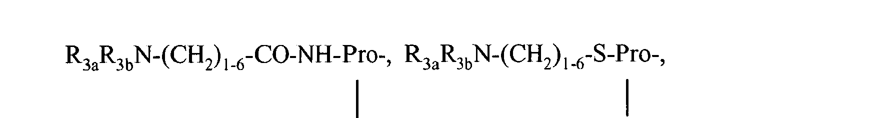 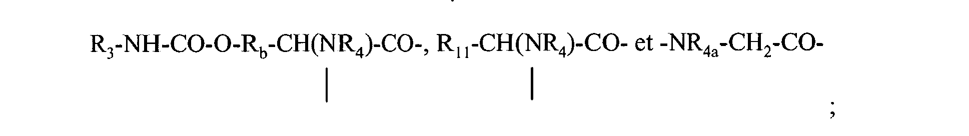
R₃ is NR₈R₉-C₂₋₆alkylene, guanidino-C₂₋₆alkylene or C₂₋₆alkylene-COOH, R₃ₐ is H, C₁₋₄alkyl or has, independently, one of the meanings given for R₃, R_{3b} is H or C₁₋₄ alkyl, Rₐ is OH or NR₅R₆, R_{b} is -(CH₂)₁₋₃- or -CH(CH₃)-, R₄ is H or CH₃, R₄ₐ is benzyl optionally substituted on the aromatic ring, each of R₅ and R₆ is independently H, C₁₋₄alkyl, ω-amino-C₁₋₄alkylene, ω-hydroxy-C₁₋₄alkylene or acyl, R₇ is a direct bond or C₁₋₆alkylene, each of R₈ and R₉ is independently H, C₁₋₄alkyl, ω-hydroxy-C₂₋₄alkylene, acyl or CH₂OH-(CHOH)_{c}-CH₂- in which c is 0, 1, 2, 3 or 4, or R₈ and R₉ form together with the nitrogen atom to which they are attached a heterocyclic group which can include an additional heteroatom, and R₁₁ is benzyl optionally substituted on the aromatic ring, -(CH₂)₁₋₃-OH, CH₃-CH(OH)- or - (CH₂)₁₋₅-NR₅R₆, and ZZₐ is a natural or unnatural α-amino acid unit;
it being understood that X₁, X₂ and Lys each have the configuration L;
or is a pharmaceutically acceptable salt or protected form of a compound of general formula **(I)**.

5. Use according to claim 3, **characterized in that** the somatostatin agonist analogue is a compound of general formula **(II)** 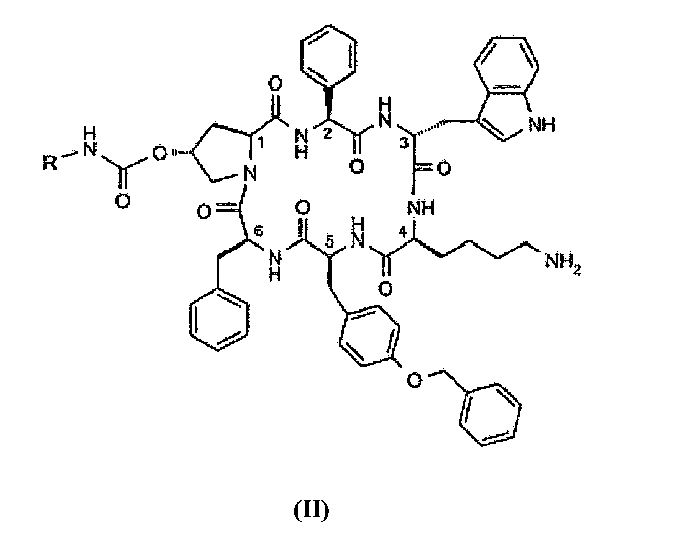 in which R is NR₁₀R₁₁-C₂₋₆alkylene or guanidine-C₂₋₆alkylene, and each of R₁₀ and R₁₁ is independently H or C₁₋₄alkyl
or is a pharmaceutically acceptable salts or a protected form of a compound of general formula **(II)**.

6. Use according to claim 3, **characterized in that** the somatostatin agonist analogue is chosen from the group comprising lanreotide, octreotide, vapreotide, SOM 230, MK-678, BIM-23190, BIM-23197, BIM-23268, PTR-3173, TT-232, the peptide of formula c[Tic-Tyr-DTrp-Lys-Abu-Phe], the KE 108 peptide of formula Tyr⁰-(cyclo-D-Dab-Arg-Phe-Phe-D-Trp-Lys-Thr-Phe) and their pharmaceutically acceptable salts and protected forms.

7. Use according to claim 6, **characterized in that** the somatostatin agonist analogue is lanreotide or one of its pharmaceutically acceptable salts.

8. Use according to one of claims 1 to 7, **characterized in that** the medicament is intended to be administered to a woman at risk of early menopause.

9. Use according to one of claims 1 to 7, **characterized in that** the medicament is intended to be administered to a woman who has an X chromosome microdeletion.

10. Use according to one of claims 1 to 7, **characterized in that** the medicament is intended to be administered to a woman who has polycystic ovaries.

11. Use according to one of claims 1 to 7, **characterized in that** the medicament is intended to be administered to a woman who is about to have, is currently having or has had chemotherapy or irradiation.

12. Use of somatostatin or one of its agonist analogues in toxicology tests relating to another compound in order to determine the presence or the absence of an effect of acceleration of follicle growth caused by said other compound.

13. Use of a somatostatin antagonist analogue for preparing a medicament intended to accelerate the start of growth of the quiescent follicles in non-menopausal women.

14. Use according to claim 13, **characterized in that** the somatostatin antagonist analogue is chosen from the peptides of general formula **(III)**
A¹-cyclo {D-Cys-A²-D-Trp-A³-A⁴-Cys}-A⁵-Y **(III)**
in which:
A¹ is an optionally substituted aromatic α-amino acid;
A² is an optionally substituted aromatic α-amino acid;
A³ is Dab, Dap, Lys or Orn;
A⁴ is β-Hydroxyvaline, Ser, Hser, or Thr;
A⁵ is an optionally substituted aromatic D- or L- α-amino acid; and
Y¹ is OH, NH₂ or NHR¹, R¹ being (C₁₋₆) alkyl;
each optionally substituted aromatic α-amino acid being optionally substituted with one or more substituents independently chosen from the group comprising a halogen atom and the groups NO₂, OH, CN, (C₁₋₆)alkyl, (C₂₋₆)alkenyl, (C₂₋₆)alkynyl, (C₁-₆)alkoxy, Bzl, O-Bzl and NR⁹R¹⁰, R⁹ and R¹⁰ each being independently H, O, or (C₁-₆) alkyl; and
each nitrogen atom with a peptide amide bond and the amino group of A¹ being optionally substituted with a methyl group, it being understood that there is at least one such methyl group in a peptide of general formula **(III)**;
and the pharmaceutically acceptable salts and protected forms of such peptides.

15. Use according to claim 13, **characterized in that** the somatostatin antagonist analogue is chosen from the group comprising:
❖ the following peptides:
- Cpa-cyclo[D-Cys-Pal-D-Trp-N-Me-Lys-Thr-Cys]-D-Trp-NH_{2;}
- Cpa-cyclo[D-Cys-Tyr-D-Trp- N-Me-Lys-Thr-Cys]-Nal-NH_{2;}
- Cpa-cyclo[D-Cys-Pal-D-Trp- N-Me-Lys-Thr-Cys]-Nal-NH₂;
❖ the peptide known by the code name AC-178,335;
❖ the octapeptide known by the code name ODN-8;
❖ the peptide known by the code name SB-710411;
❖ the peptide known by the code name BlM-23056;
❖ the compound known by the code name BN-81674;
❖ the compound known by the code name SRA-880;
and their pharmaceutically acceptable salts and protected forms.

16. Use of a somatostatin antagonist analogue in order to support *in vitro* follicle development.

17. Use of a somatostatin antagonist analogue in toxicology tests relating to another compound in order to determine the presence or the absence of an effect of slowing the follicle growth caused by said other compound.

## Patentansprüche

1. Verwendung von Somatostatin oder eines seiner Agonisten-Analoga zur Herstellung eines Medikaments, das dazu bestimmt ist, den Follikelvorrat der Eierstöcke zu regulieren und insbesondere die Erschöpfung des Follikelvorrats der Eierstöcke mit der Zeit bei der nicht menopausierten Frau zu verringern.

2. Verwendung nach Anspruch 1, **dadurch gekennzeichnet, dass** das Somatostatin zur Herstellung des Medikaments verwendet wird.

3. Verwendung nach Anspruch 1, **dadurch gekennzeichnet, dass** ein Agonisten-Analogon des Somatostatins zur Herstellung des Medikaments verwendet wird.

4. Verwendung nach Anspruch 3, **dadurch gekennzeichnet, dass** das Agonisten-Analogon des Somatostatins eine Verbindung der allgemeinen Formel (I) 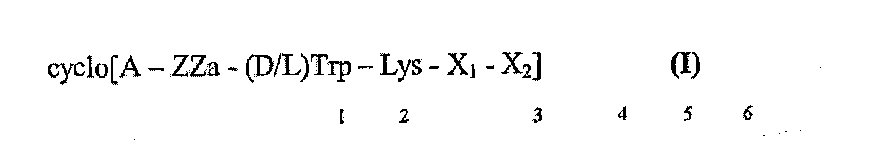 ist, in der:
X₁ ein Rest der Formel (a) oder (b) ist 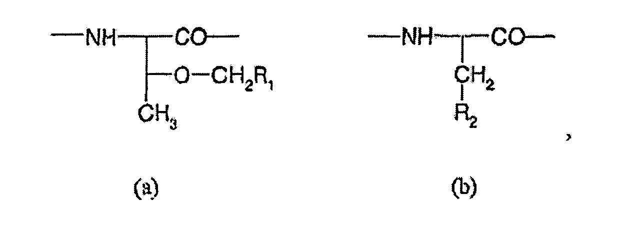 wobei R₁ jedes Mal, wenn es auftritt, unabhängig voneinander einen gegebenenfalls substituierten Phenylrest darstellt, bei dem die gegebenenfalls vorgesehenen Substituenten unabhängig voneinander aus einem Halogenatom und den Resten Methyl, Ethyl, Methoxy und Ethoxy ausgewählt sind,
R₂ darstellt: -Z₁-CH₂-R₁, -CH₂-CO-O-CH₂-R₁, 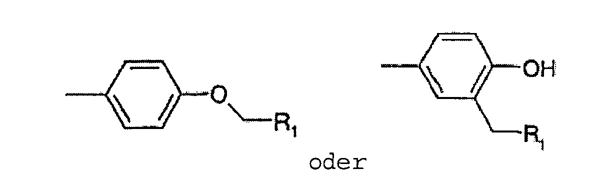
Z₁ O oder S ist;
X₂ eine α-Aminosäure mit einem aromatischen Rest an der Seitenkette C_{α} oder eine Aminosäureeinheit ist, die aus Dab, Dpr, Dpm, His, (Bzl)HyPro, Thienyl-Ala, Cyclohexyl-Ala und t-Butyl-Ala ausgewählt ist;
A ein zweiwertiger Rest ist, der ausgewählt ist aus Pro, 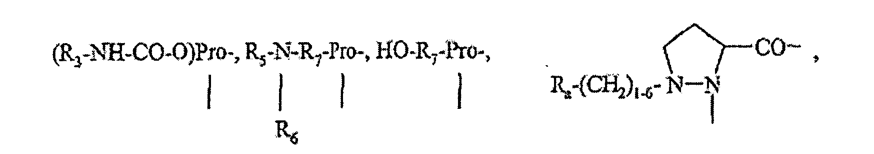 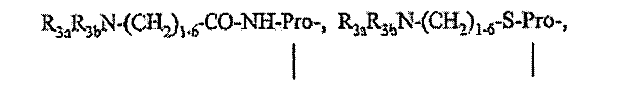 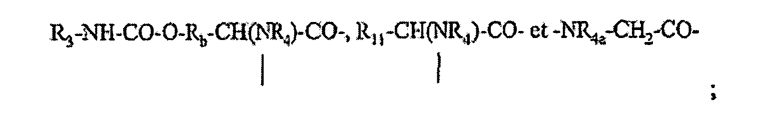
R₃ NR₈R₉-C₂₋₆-Alkylen, Guanidino-C₂₋₆-Alkylen oder C₂₋₆-Alkylen-COOH ist, R₃ₐ H, C₁₋₄-Alkyl ist oder unabhängig voneinander eine der für R₃ angegebenen Bedeutungen hat, R_{3b} H oder C₁₋₄-Alkyl ist, Rₐ OH oder NR₅R₆ ist, R_{b} - (CH₂)₁₋₃- oder -CH(CH₃)- ist, R₄ H oder CH₃ ist, R₄ₐ gegebenenfalls an dem aromatischen Ring substituiertes Benzyl ist, jeder der Reste R₅ und R₆ unabhängig voneinander H, C₁₋₄-Alkyl, ω-Amino-C₁₋₄-Alkylen, ω-Hydroxy-C₁₋₄-Alkylen oder Acyl ist, R₇ eine direkte Bindung oder C₁₋₆-Alkylen ist, wobei jeder der Reste R₈ und R₉ unabhängig voneinander H, C₁₋₄-Alkyl, ω-Hydroxy-C₂₋₄-Alkylen, Acyl oder CH₂OH-(CHOH)_{c}-CH₂- ist, worin c 0, 1, 2, 3, oder 4 ist, oder R₈ und R₉ zusammen mit dem Stickstoffatom, an das sie gebunden sind, eine heterocyclische Gruppe bilden, die ein zusätzliches Heteroatom umfassen kann, und R₁₁ gegebenenfalls an dem aromatischen Ring substituiertes Benzyl, -(CH₂)₁₋₃-OH, CH₃-CH (OH) - oder - (CH₂)₁₋₅-NR₅R₆ ist, und ZZₐ eine natürliche oder nicht natürliche α-Aminosäureneinheit ist;
wobei gilt, dass X₁, X₂ und Lys jeweils die Konfiguration L haben;
oder ein pharmazeutisch annehmbares Salz oder eine geschützte Form einer Verbindung der allgemeinen Formel (I) ist.

5. Verwendung nach Anspruch 3, **dadurch gekennzeichnet, dass** das Agonisten-Analogon des Somatostatins eine Verbindung der allgemeinen Formel (II) 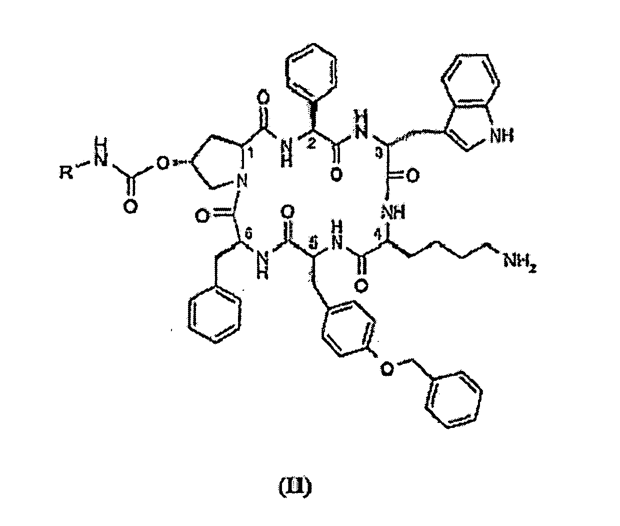 ist, in der R NR₁₀R₁₁-C₂₋₆-Alkylen oder Guanidin-C₂₋₆-Alkylen ist und jeder der Reste R₁₀ und R₁₁ unabhängig voneinander H oder C₁₋₄-Alkyl ist,
oder ein pharmazeutisch annehmbares Salz oder eine geschützte Form einer Verbindung der allgemeinen Formel (II) ist.

6. Verwendung nach Anspruch 3, **dadurch gekennzeichnet, dass** das Agonistenanalogon des Somatostatins aus der Gruppe ausgewählt ist, die besteht aus Lanreotid, Octreotid, Vapreotid, SOM 230, MK-678, BIM-23190, BIM-23197, BIM-23268, PTR-3173, TT-232, Peptid der Formel c[Tic-Tyr-DTrp-Lys-Abu-Phe], Peptid KE 108 der Formel Tyr⁰-(cyclo-D-Dab-Arg-Phe-Phe-D-Trp-Lys-Thr-Phe) und pharmazeutisch annehmbaren Salzen und geschützten Formen von diesen.

7. Verwendung nach Anspruch 6, **dadurch gekennzeichnet, dass** das Agonistenanalogon des Somatostatins Lanreotid oder eines seiner pharmazeutisch annehmbaren Salze ist.

8. Verwendung nach einem der Ansprüche 1 bis 7, **dadurch gekennzeichnet, dass** das Medikament dazu bestimmt ist, einer Frau mit einem Risikofaktor für vorzeitige Menopause verabreicht zu werden.

9. Verwendung nach einem der Ansprüche 1 bis 7, **dadurch gekennzeichnet, dass** das Medikament dazu bestimmt ist, einer Frau verabreicht zu werden, bei der eine Mikrodeletion des Chromosoms X vorliegt.

10. Verwendung nach einem der Ansprüche 1 bis 7, **dadurch gekennzeichnet, dass** das Medikament dazu bestimmt ist, einer Frau mit polyzystischen Eierstöcken verabreicht zu werden.

11. Verwendung nach einem der Ansprüche 1 bis 7, **dadurch gekennzeichnet, dass** das Medikament dazu bestimmt ist, einer Frau vor, während oder nach einer Chemotherapie oder Bestrahlung verabreicht zu werden.

12. Verwendung von Somatostatin oder eines seiner Agonistenanaloga in sich auf eine andere Verbindung beziehenden toxikologischen Tests zur Bestimmung des Vorhandenseins oder des Fehlens eines durch diese andere Verbindung bewirkten Effekts der Beschleunigung des Follikelwachstums.

13. Verwendung eines Antagonisten-Analogon des Somatostatins zur Herstellung eines Medikaments, das dazu bestimmt ist, den Eintritt des Wachstums der ruhenden Follikel bei der nicht menopausierten Frau zu beschleunigen.

14. Verwendung nach Anspruch 13, **dadurch gekennzeichnet, dass** das Antagonisten-Analogon des Somatostatins aus den Peptiden der allgemeinen Formel (III) ausgewählt ist
A¹-cyclo{D-Cys-A²-D-Trp-A³-A⁴-Cys}-A⁵-Y¹ **(III)**
in der:
A¹ eine gegebenenfalls substituierte aromatische α-Aminosäure ist;
A² eine gegebenenfalls substituierte aromatische α-Aminosäure ist;
A³ Dab, Dap, Lys oder Orn ist;
A⁴ β-Hydroxyvalin, Ser, Hser oder Thr ist;
A⁵ eine gegebenenfalls substituierte aromatische D- oder L-α-Aminosäure ist; und
Y¹ OH, NH₂ oder NHR¹ ist, wobei R¹ (C₁₋₆)-Alkyl ist;
wobei jede gegebenenfalls substituierte aromatische α-Aminosäure gegebenenfalls mit einem oder mehreren Substituenten substituiert ist, die unabhängig voneinander aus der Gruppe ausgewählt sind, die besteht aus einem Halogenatom und aus den Gruppen NO₂, OH, CN, (C₁₋₆) -Alkyl, (C₂₋₆)-Alkenyl, (C₂₋₆) -Alkinyl, (C₁₋₆)-Alkoxy, Bzl, O-Bzl und NR⁹R¹⁰, wobei R⁹ und R¹⁰ jeweils unabhängig voneinander H, O oder (C₁₋₆)-Alkyl sind; und jedes Stickstoffatom der Peptidamidbindung und die Aminogruppe von A¹ gegebenenfalls jeweils mit einer Methylgruppe substituiert sind, wobei gilt, dass es mindestens eine solche Methylgruppe in einem Peptid der allgemeinen Formel (III) gibt;
und die pharmazeutisch annehmbaren Salze und geschützten Formen solcher Peptide.

15. Verwendung nach Anspruch 13, **dadurch gekennzeichnet, dass** das Antagonisten-Analogon des Somatostatins aus der Gruppe ausgewählt ist, die besteht aus:
❖ den folgenden Peptiden:
- Cpa-cyclo[D-Cys-Pal-D-Trp-N-Me-Lys-Thr-Cys]-D-Trp-NH₂;
- Cpa-cyclo[D-Cys-Tyr-D-Trp-N-Me-Lys-Thr-Cys]-Nal-NH₂;
- Cpa-cyclo[D-Cys-Pal-D-Trp-N-Me-Lys-Thr-Cys]-Nal-NH₂;
❖ dem unter dem Codenamen AC-178,335 bekannten Peptid;
❖ dem unter dem Codenamen ODN-8 bekannten Octapeptid;
❖ dem unter dem Codenamen SB-710411 bekannten Peptid;
❖ dem unter dem Codenamen BIM-23056 bekannten Peptid;
❖ der unter dem Codenamen BN-81674 bekannten Verbindung;
❖ der unter dem Codenamen SRA-880 bekannten Verbindung;
und ihren pharmazeutisch annehmbaren Salzen und geschützten Formen.

16. Verwendung eines Antagonisten-Analogons des Somatostatins zur Unterstützung der Follikelentwicklung *in vitro*.

17. Verwendung eines Antagonisten-Analogons des Somatostatins in eine andere Verbindung betreffenden toxikologischen Tests zur Bestimmung des Vorhandenseins oder des Fehlens eines durch diese andere Verbindung verursachten Effekts der Verlangsamung des Follikelwachstums.
